(19) **Europäisches Patentamt**

**European Patent Office**

**Office européen des brevets**

(11) Veröffentlichungsnummer : **0 382 248 B1**

(12) **EUROPÄISCHE PATENTSCHRIFT**

(45) Veröffentlichungstag der Patentschrift :
**22.04.92 Patentblatt 92/17**

(21) Anmeldenummer : **90102590.8**

(22) Anmeldetag : **09.02.90**

(51) Int. Cl.⁵ : **B65C 1/02,** B65B 61/20

(54) **Verfahren und Vorrichtung zum Zuordnen von Bindeetiketten zu Strauchpflanzen.**

(30) Priorität : **10.02.89 DE 3904044**

(43) Veröffentlichungstag der Anmeldung :
**16.08.90 Patentblatt 90/33**

(45) Bekanntmachung des Hinweises auf die
Patenterteilung :
**22.04.92 Patentblatt 92/17**

(84) Benannte Vertragsstaaten :
**AT BE CH DE DK ES FR GB GR IT LI LU NL SE**

(56) Entgegenhaltungen :
**US-A- 3 549 459**
**US-A- 4 351 679**
**US-A- 4 605 459**

(73) Patentinhaber : **W. KORDES'SÖHNE**
**ROSENSCHULEN GMBH & CO. KG**
**Rosenstrasse 54**
**W-2206 Klein Offenseth-Sparrieshoop (DE)**

(72) Erfinder : **Hoppe, Peter**
**Rosenstrasse 19h**
**W-2206 Klein Offenseth-Sparrieshoop (DE)**
Erfinder : **Helms, Bernd**
**Schlottweg 2**
**W-2206 Klein Offenseth-Sparrieshoop (DE)**
Erfinder : **Peters, Reiner**
**Wohlt 7**
**W-2201 Ellerhoop (DE)**

(74) Vertreter : **Goetz, Rupert, Dipl.-Ing. et al**
**Wuesthoff & Wuesthoff Patent- und**
**Rechtsanwälte Schweigerstrasse 2**
**W-8000 München 90 (DE)**

Anmerkung : Innerhalb von neun Monaten nach der Bekanntmachung des Hinweises auf die Erteilung des europäischen Patents kann jedermann beim Europäischen Patentamt gegen das erteilte europä- ische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patent- übereinkommen).

**Beschreibung**

Die Erfindung betrifft ein Verfahren und eine Vorrichtung zum Zuordnen von Bindeetiketten an Verpackungen von Strauchpflanzen.

Pflanzen, u.a. Rosen- und andere Strauchpflanzen, werden immer häufiger über Supermärkte und andere Verkaufsstellen vertrieben, die nicht unbedingt über gärtnerisch geschultes Personal verfügen. Es ist deshalb wichtig, daß die zum Zwecke des Versandes und Verkaufs meist einzeln verpackten Pflanzen an gut sichtbarer Stelle der Verpackung mit einem Etikett versehen sind, das alle für den Käufer erforderlichen Angaben über Eigenschaften und Behandlung der betreffenden Pflanze in schriftlicher und/oder bildlicher Form enthält. Unabhängig davon kann es wünschenswert sein, der Pflanze ein Bindeetikett beizugeben, das auch nach dem Einpflanzen mit ihr verbunden bleibt. Solche Bindeetiketten können neben dem Pflanzennamen beispielsweise Angaben enthalten, die für die Durchführung von Lizenzverträgen von Bedeutung sind.

Grundsätzlich ist es möglich, Bindeetiketten beim Verpacken von Pflanzen in die Verpackungen einzulegen. Andererseits könnte man Bindeetiketten schon bei oder vor dem Verpacken an Pflanzentriebe anbinden, die dann aus der Verpackung herausragen. In beiden Fällen ist es aber mühsam, bei einer Endkontrolle der verpackten Pflanzen festzustellen, ob jeder Pflanze ein und nur ein Bindeetikett beigegeben worden ist.

Der Erfindung liegt deshalb die Aufgabe zugrunde, Pflanzenverpackungen in eindeutiger und arbeitssparender Weise Bindeetiketten zuzuordnen.

Die Aufgabe ist, soweit sie das Verfahren zum Anbringen von Etiketten betrifft, erfindungsgemäß dadurch gelöst, daß ein Bindeetikett, an dem ein Bindedraht zum späteren Anbringen an einer Pflanze befestigt ist, mittels eines den Bindedraht abdeckenden Klebeetiketts an einer Pflanzenverpackung befestigt wird, aus der die Pflanze am Pflanzort herausgenommen wird.

Das Befestigen des Bindeetiketts mittels des Klebeetiketts kann im einfachsten Fall manuell geschehen, indem eine Person mit einer Hand ein Bindeetikett an die Pflanzenverpackung anlegt und mit der anderen Hand das Klebeetikett über den Bindedraht klebt. Die Haftung des Klebeetiketts an der Pflanzenverpackung wird durch den dazwischenliegenden Bindedraht nicht beeinträchtigt und ist bei Klebeetiketten üblicher Größe selbst dann noch bei weitem ausreichend, wenn das Klebeetikett auch auf einen Teil des Bindeetiketts aufgeklebt wird, an dem der Bindedraht befestigt ist. Dieses Überkleben eines Teils des Bindeetiketts hat den Vorteil, daß das Bindeetikett ebenso wie das Klebeetikett flach an der Pflanzenverpackung anliegend gehalten wird, so daß beispielsweise mit optoelektronischen Einrichtungen bekannter Art in einer Verpackungsanlage zuverlässig nachgeprüft werden kann, ob jede Pflanzenverpackung neben dem Klebeetikett mit einem und nur mit einem Bindeetikett versehen worden ist.

Soweit die beschriebene Aufgabe eine Vorrichtung betrifft, ist sie erfindungsgemäß gelöst durch
– mindestens einen Etikettenbehälter, in dem Bindeetiketten gestapelt und in einer Lage bereitgehalten werden, in der ihre Bindedrähte in gleicher Richtung angeordnet sind,
– einen Stempel zum Entnehmen eines Bindeetiketts aus dem Etikettenbehälter und Andrücken des Bindeetiketts an die zu etikettierende Pflanzenverpackung und
– einen Klebeetikettenspender zum Andrücken eines Klebeetiketts an den Bindedraht und die Pflanzenverpackung.

Vorteilhafte Weiterbildungen ergeben sich aus den Unteransprüchen.

Ein Ausführungsbeispiel der Erfindung wird im folgenden anhand schematischer Zeichnungen einer Anlage zum Verpacken und Etikettieren ballierter Strauchpflanzen, insbes. Rosenpflanzen, mit weiteren Einzelheiten erläutert.

Es zeigen:

Fig. 1 die Draufsicht der Anlage,
Fig. 2 die Seitenansicht in Richtung des Pfeils II in Fig. 1,

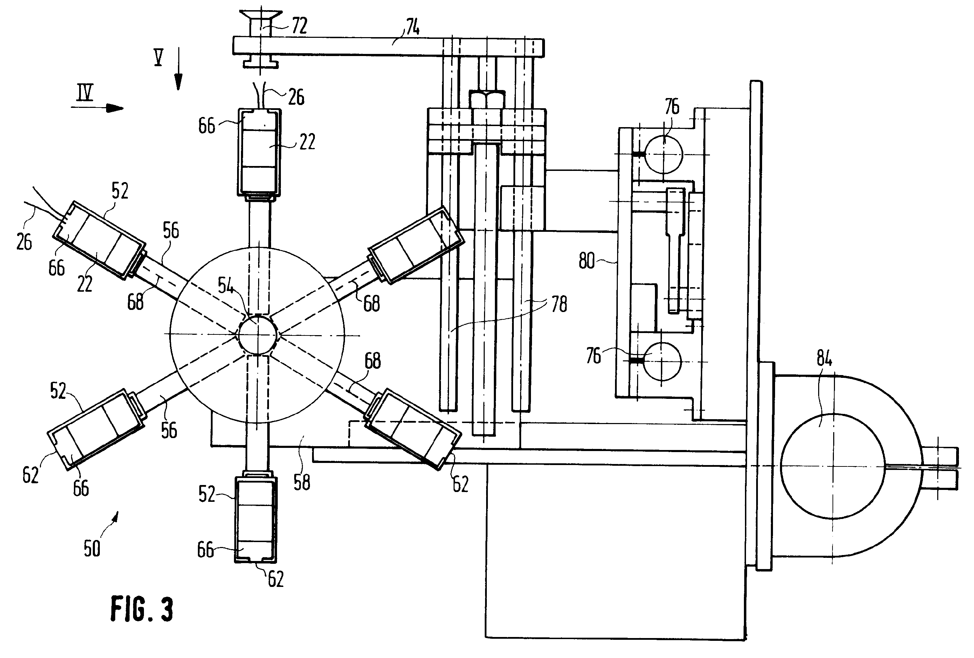

Fig. 3 die vergrößerte Teildraufsicht im Bereich des Pfeils III in Fig. 2,

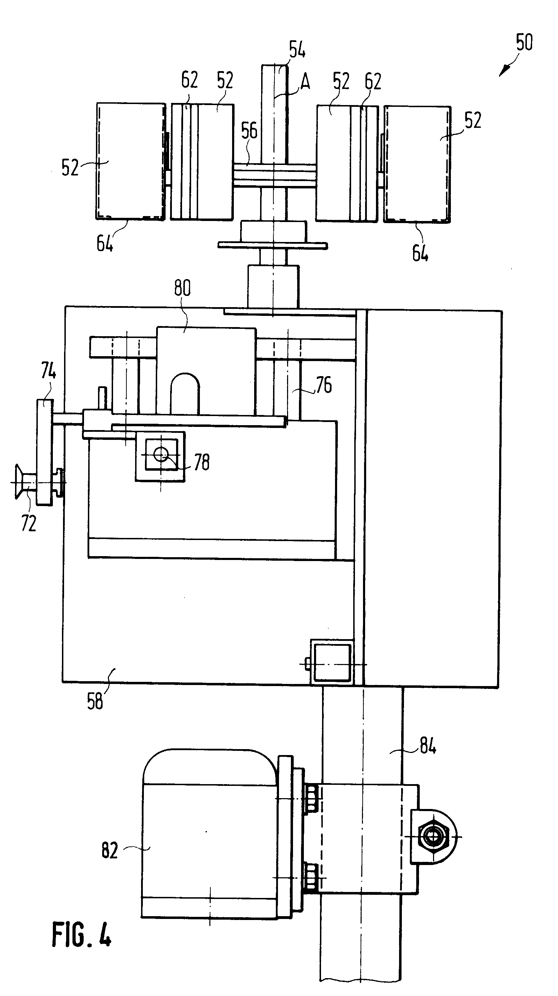

Fig. 4 die Ansicht in Richtung des Pfeils IV in Fig. 3,

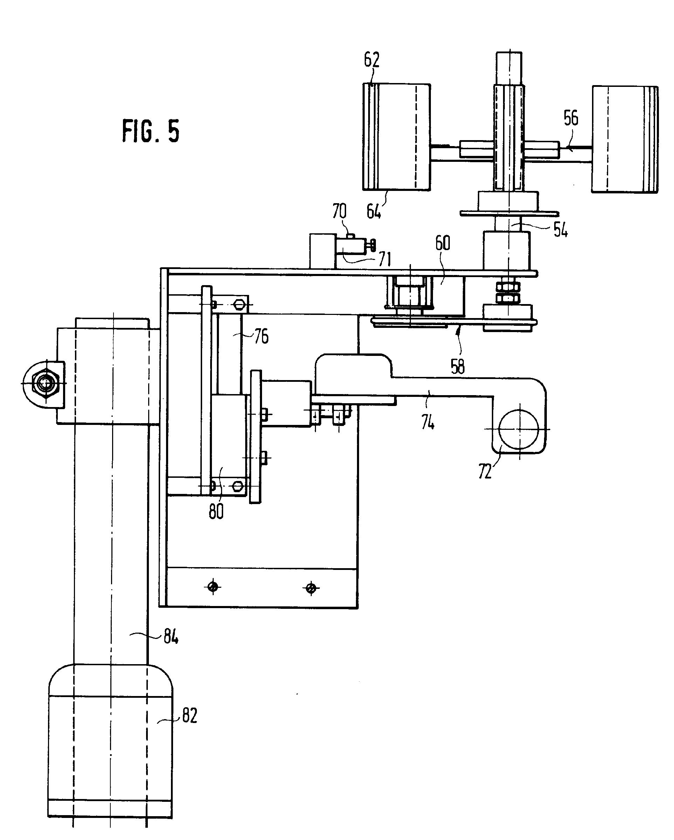

Fig. 5 die Ansicht in Richtung des Pfeils V in Fig. 3, und
Fig. 6 den vergrößerten Teilschnitt III-III in Fig. 1.

Die dargestellte Anlage dient dazu, ballierte strauchartige Pflanzen 10 in je einen Beutel 12 aus schrumpffähiger Folie zu stecken und den Beutel im Bereich zwischen Pflanzentrieben und Wurzelballen durch örtlich begrenztes Schrumpfen zu verschließen.

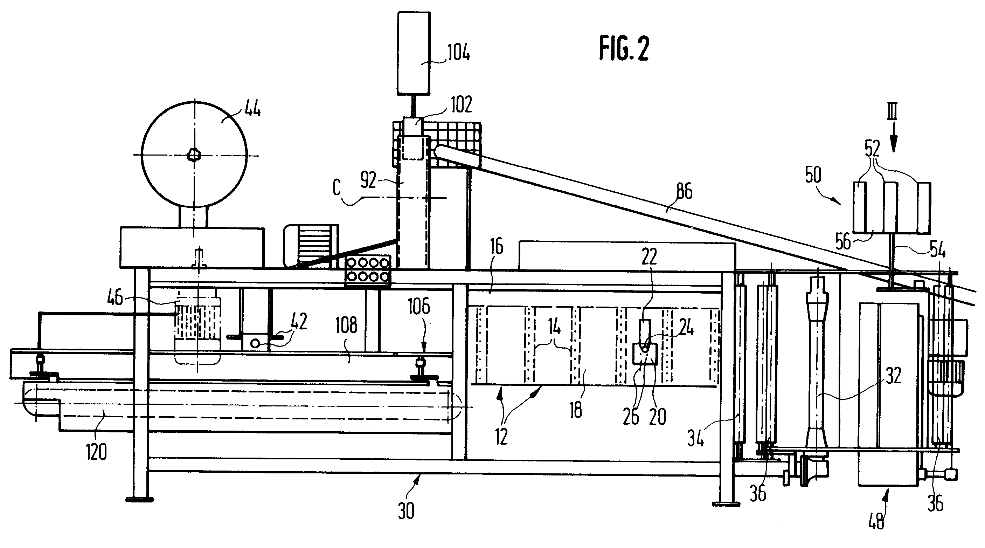

Die Beutel 12 sind, noch miteinander zusammenhängend, in einer schlauchförmigen Folienbahn ausgebildet, die in einer senkrechten Längsebene aufrechtstehend waagerecht durch die Anlage hindurchgefördert wird und an ihrer Oberseite aufgeschlitzt ist. Die einzelnen Beutel 12 sind durch senkrechte Schweißnähte 14 begrenzt, die unterhalb der durch das Aufschlitzen voneinander getrennten oberen Beutelrandbereiche 16 enden. Jeder Beutel 12 hat eine dem Betrachter der Fig. 2 zugewandte Sichtfläche 18, auf die ein Klebeetikett 20 aufgeklebt werden soll.

Jedem Beutel 12 soll ferner ein Bindeetikett 22 beigegeben werden, das an der Pflanze 10 befestigt werden kann, wenn diese am Pflanzort aus dem Beutel 12 herausgenommen wird. Jedes Bindeetikett 22 hat eine Öse 24, in der ein Bindedraht 26 befestigt ist. Zum Befestigen des Bindeetiketts 22 am Beutel 12 soll das Klebeetikett 20 über den Bindedraht 26 und sicherheitshalber auch über den Bereich der Öse 24 geklebt werden. Durch Schrumpfen soll der Beutel 12 schließlich einen Hals 28 bilden; unterhalb und auch oberhalb des Halses 28 soll der Beutel 12 aber vom Schrumpfen nicht oder nur verhältnismäßig wenig betroffen werden.

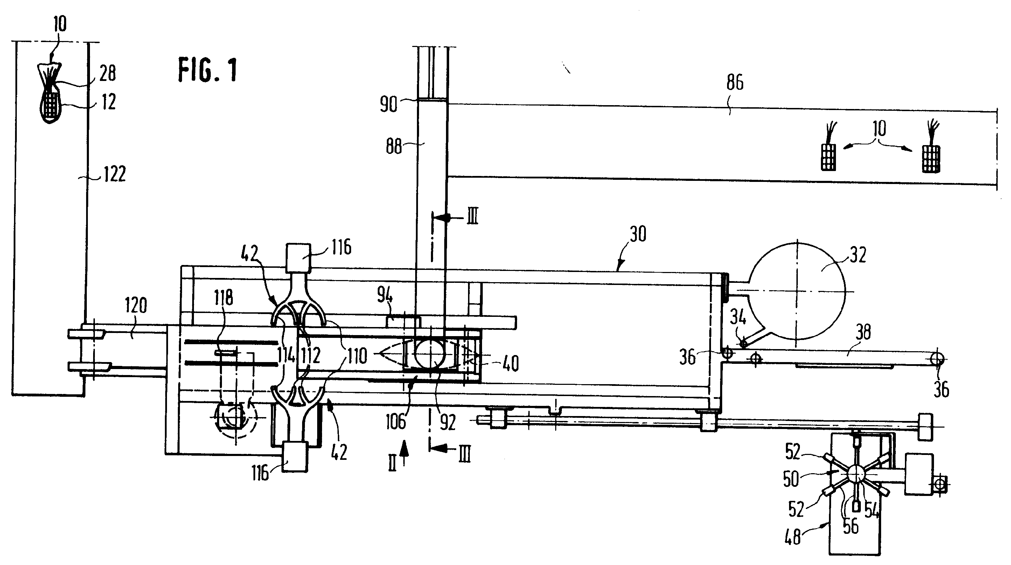

Die dargestellte Anlage hat ein langgestrecktes Maschinengestell 30, an dessen Anfang, in Fig. 1 und 2 rechts, eine senkrechte Abwickelhaspel 32 für die Folienbahn gelagert ist, in der die Beutel 12 ausgebildet sind. Ausgehend von der Haspel 32 läuft die Folienbahn schrittweise über eine Spannrolle 34 sowie über Umlenkrollen 36 und an einer senkrechten Stützfläche 38 entlang zu einem senkrechten rohrförmigen Spreizkörper 40, der in Draufsicht gemäß Fig. 1 die Form eines an beiden Enden spitzen Schiffsrumpfes hat. Der Spreizkörper 40 greift zwischen die oberen Beutelrandbereiche 16 ein und spreizt sie nach jedem Förderschritt der Folienbahn oberhalb eines Beutels 12 soweit auseinander, daß der betreffende Beutel 12 sich zum Aufnehmen einer ballierten Pflanze 10 öffnet.

Die Beutel 12, die noch zusammenhängen, nun aber mit je einer Pflanze 10 gefüllt sind, laufen anschließend zum Schrumpfen ihres Halses 28 zwischen zwei Düsengruppen 42 hindurch. Oberhalb des hinteren, in Fig. 1 und 2 linken Endes des Maschinengestells 30 ist eine Aufwickelhaspel 44 zum Aufwickeln der von den Beuteln 12 abgerissenen oberen Beutelrandbereiche 16 angeordnet. Für die Bewegung der die Beutel 12 bildenden Folie durch die Anlage hindurch und zum Drehen der Aufwickelhaspel 44 ist ein gemeinsamer Motor 46 vorgesehen.

Am Anfang der Anlage, in Fig. 1 und 2 rechts, sind ein Klebeetikettenspender 48 und, darüber, ein Bindeetikettenspender 50 angeordnet. Der Klebeetikettenspender 48 ist als solcher von bekannter Bauart und deshalb nicht näher dargestellt. Der Bindeetikettenspender 50 hat im dargestellten Beispiel sechs Etikettenbehälter 52, die am Ende je eines von einer senkrechte Welle 54 radial wegragenden Arms 56 befestigt sind. Die Welle 54 ist über ein Getriebe 58 von einem Schrittmotor 60 derart antreibbar ist, daß jeweils einer der Etikettenbehälter 52 eine genau festgelegte Entnahmestellung solange einnimmt, bis er vollständig geleert ist.

In jedem Etikettenbehälter 52 ist, an dessen vom zugehörigen Arm 56 abgewandter Schmalseite, ein senkrechter Schlitz 62 ausgebildet, der in eine Bodenöffnung 64 mündet. In gefülltem Zustand enthält jeder Etikettenbehälter 52 eine Vielzahl flach aufeinanderliegender Bindeetiketten 22, deren Bindedrähte 26 durch den Schlitz 62 nach außen ragen. Ferner enthält jeder Etikettenbehälter 52 ein in seinem senkrechten Schlitz 62 geführtes Gewicht 66 aus Metall, das die Bindeetiketten 22 belastet und über deren Ösen 24, die ebenfalls aus Metall bestehen, mit den zugehörigen, gleichfalls metallischen oder metallisierten Bindedrähten 26 in elektrisch leitender Verbindung steht. Die Etikettenbehälter 52 selbst sind elektrisch nichtleitend.

Jedes der Gewichte 66 ist über eine flexible Leitung 68 mit einem Pol einer elektrischen Stromquelle verbunden, deren zweiter Pol über eine nicht dargestellte Schaltung an einen Kontakt 70 angeschlossen ist. Der Bindedraht 26 des untersten Bindeetiketts 22 in dem Etikettenbehälter 52, der die Entnahmestellung einnimmt, liegt auf dem Kontakt 70 auf, wodurch ein elektrischer Stromkreis geschlossen wird.

Dieser Stromkreis wird unterbrochen, sobald das letzte Bindeetikett 22 in dem betreffenden Etikettenbehälter 52 verbraucht ist. Dadurch wird ein Signal erzeugt, das die Welle 54 sich solange drehen läßt, bis ein noch nicht geleerter Etikettenbehälter 52 die Entnahmestellung erreicht hat. Der Kontakt 70 ist am Ende einer Schiene 71 angeordnet, die aus elektrisch nichtleitendem Werkstoff besteht und bogenförmig derart um die Achse A gekrümmt ist, daß beim Drehen der Welle 54 die Bindedrähte 26 dieses noch nicht geleerten Etikettenbehälters 52 auf die Schiene 71 auflaufen, bis sie schließlich den Kontakt 70 erreichen. Dadurch wird der genannte Stromkreis wieder geschlossen und infolgedessen die Drehung um die Achse A unterbrochen.

Zum Entnehmen jeweils eines Bindeetiketts 22 aus dem in Entnahmestellung stehenden Etikettenbehälter 52, und zum Andrücken dieses Bindeetiketts 22 an die Sichtfläche 18 jeweils eines Beutels 12 weist der Bindeetikettenspender 50 einen Stempel 72 auf, der im dargestellten Beispiel als Saugnapf ausgebildet und über ein nicht dargestelltes Ventil an eine Vakuumquelle angeschlossen ist. Der Stempel 72 ist am Ende eines Arms 74 befestigt, der längs senkrechter Führungen 76 und waagerechter Führungen 78 verschiebbar und durch eine Kulisse 80 derart gesteuert ist, daß der Stempel 72 sich bei jedem Arbeitszyklus von unten her dem in Entnahmestellung stehenden Etikettenbehälter 52 nähert, das unterste Bindeetikett 22 durch die Bodenöffnung 64 nach unten herauszieht, um 90° in eine Hochkantstellung schwenkt und an die Sichtfläche 18 des betreffenden Beutels 12 andrückt. Unmittelbar darauf klebt der Klebeetikettenspender 48 an die Sichtfläche 18 ein Klebeetikett 20 an, das den Bindedraht 26 und die Öse 24 des Bindeetiketts 22 überdeckt, so daß dieses am Beutel 12 sicher befestigt wird.

Der Arm 74 ist von einem Motor 82 antreibbar, der an einer auch die Welle 54 und deren Lagerung tragenden Säule 84 befestigt ist. Der Motor 82 ist von Lichtschranken oder anderen Sensoren gesteuert, die sicherstellen, daß beide Etikettenspender 48 und 50 jeweils dann tätig werden, wenn ein Beutel 12 den Arbeitsbereich des Stempels 72 erreicht hat.

Zum Heranschaffen der Pflanzen 10, die in je einen Beutel 12 gesteckt werden sollen, ist ein Längsförderer 86, beispielsweise in Gestalt eines Förderbandes vorgesehen, der rampenartig zu einem Querförderer 88 hin ansteigt und auf diesen die Pflanzen 10 in Abständen hintereinander fallen läßt. Der Querförderer 88 ist im dargestellten Beispiel ein oben offener Kanal, in dem jeweils eine Pflanze 10 von einem beispielsweise pneumatisch hin- und herbewegbaren Schieber 90 radial in Richtung auf die senkrechte Achse B eines Tauchrohrs 92 verschiebbar ist.

Das Tauchrohr 92 ist gleichachsig mit dem rohrförmigen Spreizkörper 40 angeordnet und von einem Antrieb 94, beispielsweise einer pneumatischen Kolbenzylindereinheit, auf- und abbewegbar zwischen einer Übernahmestellung, in der das Tauchrohr 92 von oben her nur in den Spreizkörper 40 hineinragt, und einer unteren Endstellung, in der das Tauchrohr weit in den vom Spreizkörper 40 offengehaltenen Beutel 12 hineinragt, gewünschtenfalls nahezu bis zu dessen Boden.

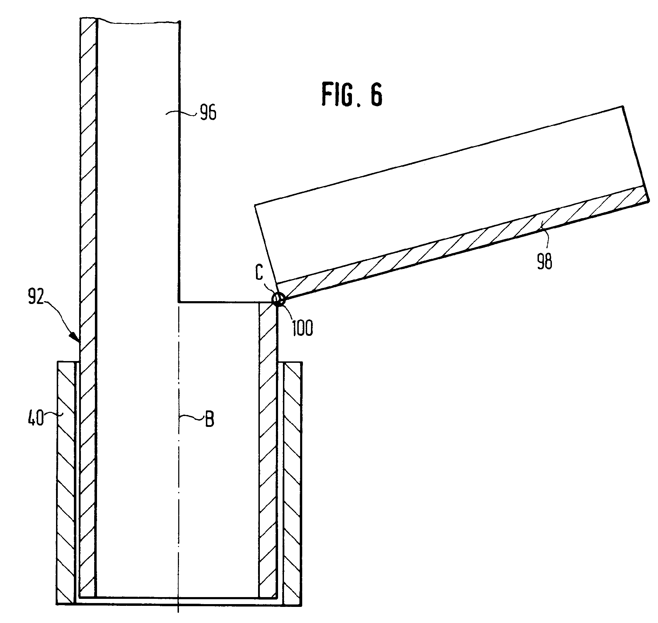

Das Tauchrohr 92 hat in seinem oberen Bereich einen halbrohrförmigen Abschnitt 96, dem eine ebenfalls halbrohrförmige Förderrinne 98 zugeordnet ist. Diese Förderrinne 98 ist an ihrem unteren Rand durch ein Gelenk 100 mit waagerechter Schwenkachse C mit dem Tauchrohr 92 verbunden. In der Übernahmestellung des Tauchrohrs 98, die in Fig. 6 abgebildet ist, mündet die Förderrinne 98 schräg in das Tauchrohr 98, so daß dieses eine Pflanze 10 vom Querförderer 88 übernehmen kann. Die Förderrinne 98 ist derart geführt, daß sie den halbrohrförmigen Abschnitt 96 zu einem vollständig geschlossenen Rohrabschnitt ergänzt, wenn das Tauchrohr 92 abgesenkt wird. Die Pflanze 10 ist infolgedessen gezwungen, an der Abwärtsbewegung des Tauchrohrs 92 teilzunehmen und kann in ihm nur mehr nach unten rutschen.

Das Abwärtsrutschen der Pflanze innerhalb des in der beschriebenen Weise seitlich geschlossenen Tauchrohrs 92 wird durch Trägheitskräfte gefördert, wenn die Abwärtsbewegung des Tauchrohrs mehr oder weniger abrupt beendet wird. Dies kann genügen, um die Pflanze 10 im Tauchrohr 92 soweit nach unten rutschen zu lassen, daß der Wurzelballen sich auf den Boden des Beutels 12 aufsetzt, in den das Tauchrohr eingetaucht ist. Sicherheitshalber ist jedoch dem Tauchrohr 92 ein kolbenartiger Stempel 102 zugeordnet, der normalerweise eine Ruhestellung im oberen Endbereich des halbrohrförmigen Abschnittes 96 oder sogar oberhalb davon einnimmt. Der Stempel 102 ist mit einer Kolbenzylindereinheit 104 verbunden, die ihn während oder unmittelbar nach dem Absenken des Tauchrohrs 92 abwärtsbewegt, so daß der Stempel 102 die Pflanze 10 mit Sicherheit soweit nach unten schiebt, bis ihr Wurzelballen auf dem Boden des Beutels 12 steht.

Unterhalb des Spreizkörpers 40 ist eine Haltevorrichtung 106 angeordnet, deren Aufgabe es ist, den Beutel 12 und die in ihn eingebrachte Pflanze 10 festzuhalten, vorzugsweise an ihrem Wurzelballen, während das Tauchrohr 92 wieder nach oben in seine Übernahmestellung bewegt wird. Im dargestellten Beispiel hat die Haltevorrichtung 106 ein Paar Backen 108, die beispielsweise pneumatisch zueinander hin und voneinander weg bewegbar sind.

Sobald das Tauchrohr 92 aus dem soeben gefüllten Beutel 12 zurückgezogen worden ist, wird das gesamte Folienband, an dem die Beutel ausgebildet sind, um einen Schritt weiterbewegt. Dadurch gelangt der soeben gefüllte Beutel mit seinem in Fig. 1 und 2 linken Rand in den Einwirkungsbereich je einer vorderen Düse 110 der beiden Düsengruppen 42, so daß aus diesen ausströmende Heißluft beginnt, den Hals 28 zum Schrumpfen zu bringen. Nach dem nächsten Förderschritt steht der genannte Beutel 12 genau zwischen zwei Paar mittleren Düsen 112; die aus diesen ausströmende Heißluft setzt die Schrumpfung am Hals 28 verstärkt fort. Nach einem weiteren Förderschritt steht der in Fig. 1 und 2 rechte Randbereich des Halses 28 zwischen hinteren Düsen 114 der beiden Düsengruppen 42, so daß die auf den Hals 28 konzentrierte Schrumpfung vollendet wird.

Die beiden Düsengruppen 42 werden von je einem Gebläse 116 immer dann mit Heißluft versorgt, wenn ein Beutel 12 in ihren Einwirkungsbereich gelangt ist. Eine sorgfältige Wärmeisolation der Düsengruppen 42 verhindert eine übermäßige Abkühlung in den Intervallen, in denen die Gebläse 116 stillstehen.

Förderstromabwärts von den Düsengruppen 42 werden die Beutelrandbereiche 16 von der entsprechend perforierten oder anderweitig vorbereiteten Folienbahn abgerissen und von der Aufwickelhaspel 44 aufgewickelt. Gleichzeitig oder unmittelbar danach werden die gefüllten und an ihrem Hals 28 geschrumpften Beutel mittels einer Trennvorrichtung 118 voneinander getrennt und von einem Längsförderer 120 sowie schließlich von einem Querförderer 122 weitergefördert.

**Patentansprüche**

1. Verfahren zum Zuordnen von Bindeetiketten an Verpackungen von Strauchpflanzen, dadurch **gekennzeichnet,** daß ein Bindeetikett (22), an dem ein Bindedraht (26) zum späteren Anbringen an einer Pflanze (10)

EP 0 382 248 B1

befestigt ist, mittels eines den Bindedraht (26) abdeckenden Klebeetiketts (20) an einer Pflanzenverpackung (12) befestigt wird, aus der die Pflanze (10) am Pflanzort herausgenommen wird.

2. Verfahren nach Anspruch 1, dadurch **gekennzeichnet,** daß das Bindeetikett (22) samt seinem Bindedraht (26) an die Pflanzenverpackung (12) angelegt wird und dann auf diese sowie auf den Bindedraht (26) das Klebeetikett (20) aufgeklebt wird, ehe die Pflanze (10) in die Pflanzenverpackung (12) eingebracht wird.

3. Verfahren nach Anspruch 1 oder 2, dadurch **gekennzeichnet,** daß das Klebeetikett (20) auch auf einen Teil des Bindeetiketts (22) aufgeklebt wird, an dem der Bindedraht (26) befestigt ist.

4. Vorrichtung zum Durchführen des Verfahrens nach einem der Ansprüche 1 bis 3, **gekennzeichnet** durch
– mindestens einen Etikettenbehälter (52), in dem Bindeetiketten (22) gestapelt und in einer Lage bereitgehalten werden, in der ihre Bindedrähte (26) in gleicher Richtung angeordnet sind,
– einen Stempel (72) zum Entnehmen eines Bindeetiketts (22) aus dem Etikettenbehälter (52) und Andrücken des Bindeetiketts (22) an die zu etikettierende Pflanzenverpackung (12) und
– einen Klebeetikettenspender (48) zum Andrücken eines Klebeetiketts (20) an den Bindedraht (26) und die Pflanzenverpackung (12).

5. Vorrichtung nach Anspruch 4, dadurch **gekennzeichnet,** daß der Etikettenbehälter (52) einen senkrechten Schlitz (62) aufweist, durch den die Bindedrähte (22) nach außen ragen.

6. Vorrichtung nach Anspruch 4, dadurch **gekennzeichnet,** daß
– der Etikettenbehälter (52) eine Bodenöffnung (64) zum Entnehmen der Bindeetiketten (22) aufweist und
– in dem Schlitz (62) ein Gewicht (66) zum Beschweren der Bindeetiketten (22) geführt ist.

7. Vorrichtung nach Anspruch 6, dadurch **gekennzeichnet,** daß der Etikettenbehälter (52) aus Isolierstoff besteht und das Gewicht (66) aus elektrisch leitendem Werkstoff besteht, an eine elektrische Leitung (68) angeschlossen ist und bei Vorhandensein mindestens eines Bindeetiketts (22) zusammen mit dessen Bindedraht (26) und einem Kontakt (70), auf dem dieser aufliegt, einen Kontrollstromkreis schließt.

8. Vorrichtung Anspruch 7, dadurch **gekennzeichnet,** daß
– mehrere Etikettenbehälter (52) einen um eine zentrale Achse (A) schrittweise drehbaren Bindeetikettenspender (50) bilden und
– der Kontakt (70) am Ende einer im übrigen elektrisch isolierten Schiene (71) angeordnet ist, auf welche die Bindedrähte (26) beim Drehen des Bindeetikettenspenders (50) aufgleiten.

9. Vorrichtung nach einem der Ansprüche 4 bis 8, dadurch **gekennzeichnet,** daß der Stempel (72) einen Saugnapf trägt und jeweils zum Entnehmen eines Bindeetiketts (22) evakuierbar sowie zum Abgeben des Bindeetiketts (22) belüftbar ist.

10. Vorrichtung nach Anspruch 9, dadurch **gekennzeichnet,** daß der Stempel (72) aus einer senkrechten Lage, in der er zum Entnehmen je eines Bindeetiketts (22) auf- und abbewegbar ist, in eine waagerechte Lage schwenkbar ist, in der er zum Andrücken des Bindeetiketts (22) an die Pflanzenverpackung (12) hin- und herbewegbar ist.


## Claims

1. A method of assigning binding labels to packages for bushy plants, **characterized** in that a binding label (22) with a binding wire (26) attached to it for future fastening to a plant (10) is affixed to a plant package (12) by an adhesive label (20) which covers the binding wire (26), the plant (10) being taken out of the package at the place of planting.

2. The method as claimed in claim 1, characterized in that the binding label (22) together with its binding wire (26) are placed on the plant package (12) and then the adhesive label (20) is glued on the binding label and on the binding wire (26) before the plant (10) is put into the plant package (12).

3. The method as claimed in claim 1 or 2, characterized in that the adhesive label (20) is glued also on part of the binding label (22) to which the binding wire (26) is attached.

4. An apparatus for carrying out the method as claimed in one of claims 1 to 3, **characterized** by
– at least one label reservoir (52) in which binding labels (22) are stacked and held ready in a position in which their binding wires (26) are disposed in the same direction,
– a stamp (72) for withdrawing a binding label (22) from the label reservoir (52) and pressing the binding label (22) against the plant package (12) to be labelled, and
– an adhesive label dispenser (48) for pressing an adhesive label (20) against the binding wire (26) and the plant package (12).

5. The apparatus as claimed in claim 4, characterized in that the label reservoir (52) includes a vertical slot (62) through which the binding wires (22) project to the outside.

6. The apparatus as claimed in claim 4, characterized in that

– the label reservoir (52) has a bottom aperture (64) for withdrawal of the binding labels (22), and

– a weight (66) for loading the binding labels (22) is guided in the slot (62).

7. The apparatus as claimed in claim 6, characterized in that the label reservoir (52) is made of insulating material and the weight (66) is made of electrically conductive material and is connected to an electric cable (68) and, in the presence of at least one binding label (22), closes a control circuit together with the binding wire (26) thereof and with a contact (70) on which the wire lies.

8. The apparatus as claimed in claim 7, characterized in that

– a plurality of label reservoirs (52) form a binding label dispenser (50) which is rotatable in stepwise manner about a central axis (A), and

– the contact (70) is arranged at the end of an otherwise electrically insulated rail (71) on to which the binding wires (26) slide as the binding label dispenser (50) rotates.

9. The apparatus as claimed in one of claims 4 to 8, characterized in that the stamp (72) carries a suction cup and is adapted to be evacuated respectively for withdrawing a binding label (22) and to be vented for discharging the binding label (22).

10. The apparatus as claimed in claim 9, characterized in that the stamp (72) is adapted to be swung from a vertical position in which it is movable up and down to withdraw a binding label (22) each into a horizontal position in which it is movable back and forth to press the binding label (12) against the plant package (12).


## Revendications

1. Procédé pour associer des étiquettes à fil à des emballages de plantes du type arbustes, caractérisé en ce qu'on fixe une étiquette à fil (22), à laquelle est fixé un fil d'attache (26) utilisé pour la fixation ultérieure de l'étiquette à une plante (10), au moyen d'une étiquette adhésive (20), qui recouvre le fil d'attache (26), sur un emballage (12) de la plante, d'où la plante (10) est retirée sur le lieu de plantation.

2. Procédé selon la revendication 1, caractérisé en ce qu'on applique l'étiquette à fil (22) ainsi que son fil d'attache (26) à l'emballage (12) de la plante et qu'on colle ensuite l'étiquette adhésive (20) sur cet emballage ainsi que sur le fil d'attache (26), juste avant d'insérer la plante (10) dans son emballage (12).

3. Procédé selon la revendication 1 ou 2, caractérisé en ce qu'on colle l'étiquette adhésive (20) également sur une partie de l'étiquette à fil (22), à laquelle est fixé le fil d'attache (26).

4. Dispositif pour la mise en oeuvre du procédé selon l'une des revendications 1 à 3, caractérisé par

– au moins un logement à étiquettes (52), dans lequel des étiquettes à fil (22) sont empilées et maintenues disponibles dans une position dans laquelle leurs fils d'attache (26) s'étendent dans la même direction,

– un piston (72) pour prélever une étiquette à fil (22) du logement à étiquettes (52) et appliquer sous pression l'étiquette à fil (22) sur l'emballage (12) de la plante, qui doit être étiquetée, et

– un ditributeur d'étiquettes adhésives (48) destiné à appliquer sous pression une étiquette adhésive (20) sur le fil d'attache (26) et sur l'emballage (12) de la plante.

5. Dispositif selon la revendication 4, caractérisé en ce que le logement à étiquettes (52) possède une fente verticale (62), par laquelle les fils d'attache (22) font saillie extérieurement.

6. Dispositif selon la revendication 4, caractérisé en ce que

– le logement à étiquettes (52) possède une ouverture de fond (64) permettant le prélèvement des étiquettes à fil (22), et

– un poids (66) destiné à appuyer sur les étiquettes à fil (22) est guidé dans la fente (62).

7. Dispositif selon la revendication 6, caractérisé en ce que le logement à étiquettes (52) est réalisé en un matériau isolant et le poids (66) est réalisé en un matériau électriquement conducteur, est raccordé à un conducteur électrique (68) et, dans le cas de la présence d'au moins une étiquette à fil (22), ferme un circuit de contrôle, conjointement avec le fil d'attache (26) de cette étiquette et un contact (70), sur lequel s'appuie le fil d'attache.

8. Dispositif selon la revendication 7, caractérisé en ce que

– plusieurs logements à étiquettes (52) forment un distributeur d'étiquettes à fil (50), qui peut tourner pas-à-pas autour d'un axe central (A), et

– le contact (70) est disposé à l'extrémité d'un rail (71), qui est par ailleurs isolé électriquement et sur lequel les fils d'attache (26) glissent lors de la rotation du distributeur d'étiquettes à fil (50).

9. Dispositif selon l'une des revendications 4 à 8, caractérisé en ce que le piston (72) porte une tête aspirante, peut être vidé respectivement pour le prélèvement d'une étiquette à fil (22) et peut être aéré pour la délivrance de l'étiquette à fil (22).

10. Dispositif selon la revendication 9, caractérisé en ce que le piston (72) peut être amené par pivotement, depuis une position verticale, dans laquelle il peut être soulevé et abaissé pour prélever respectivement une étiquette à fil (22), dans une position horizontale, dans laquelle il peut être déplacé en va-et-vient pour appliquer l'étiquette à fil (22) sur l'emballage (12) de la plante.

FIG. 1

EP 0 382 248 B1

FIG.2

EP 0 382 248 B1

FIG. 3

FIG. 4

EP 0 382 248 B1

FIG. 5

12

FIG. 6

96

92

40

B

C

100

98